# EUROPÄISCHE PATENTANMELDUNG

(11) **EP 0 656 416 A1**
(43) Veröffentlichungstag der Anmeldung: **07.06.1995**
(21) Anmeldenummer: 94115243.1
(22) Anmeldetag: 28.09.1994
(51) Int. Cl.: C11D 1/12

(54) **Tensidkonzentrate als Basistenside für konzentrierte Flüssigformulierungen**

(30) Priorität: 02.12.1993 DE 4341050
(71) Anmelder: HÜLS AKTIENGESELLSCHAFT, D-45764 Marl (DE)
(72) Erfinder: Brock, Michael, Dr., D-46514 Schermbeck (DE); Kurtin, Manfred, Dr., D-48246 Dülmen (DE); Nacke, Rudolf, D-46284 Dorsten (DE); Schulze, Klaus, Dr., D-45721 Haltern (DE)

(57) **Zusammenfassung**

Die Erfindung betrifft die Verwendung von wasser- und lösemittelfreien Tensidkonzentraten, enthaltend Mono-, Di- bzw. Trialkanolammonium-Salze der Alkylethersulfate und/oder Alkylsulfate.

Die Tensidkonzentrate mit sehr hohem Basistensidgehalt sind homogen und so niedrigviskos, daß sie bei Temperaturen bis zu 70 °C leicht zu konzentrierten homogenen und fließfähigen Flüssigformulierungen mit Tensidgehalten von 70 % und höher weiterverarbeitet werden können.

Die konzentrierten Flüssigformulierungen werden in Wasch-, Geschirrspülmittel-, Reinigungs- und Körperreinigungsmitteln eingesetzt.

## Beschreibung

Die Erfindung betrifft die Verwendung von wasser- und lösemittelfreien Tensidkonzentraten, enthaltend Mono-, Di- bzw. Trialkanolammonium-Salze der Alkylethersulfate und/oder Alkylsulfate als Basistenside für konzentrierte Flüssigformulierungen.

Die konzentrierten Flüssigformulierungen werden im Wasch-, Geschirrspül-, Reinigungs- und Körperreinigungsmittelbereich eingesetzt.

Aufgrund ökonomischer und ökologischer Überlegungen geht der Trend im Wasch-, Geschirrspül-, Reinigungs- und Körperreinigungsmittelbereich zu immer höher konzentrierten flüssigen Tensidformulierungen, die möglichst vollständig abbaubare Tenside mit vorteilhaften ökotoxikologischen Verhalten enthalten. Hier bieten sich vor allem Alkylethersulfate sowie Alkylsulfate an, die daher in zunehmendem Maße Verwendung finden (s. P. Schöberl et al., Tenside Surfactants Detergents 25 (1988), S. 86 - 98). Die Herstellung und der Einsatz von konzentrierten flüssigen Tensidformulierungen bietet gegenüber verdünnten Rezepturen vielfältige Vorteile:
- Vergrößerung der Herstellkapazität,
- Verminderung der Abfüllkosten,
- Verringerung des zu transportierenden Gewichts,
- Verminderung des Aufkommens von Verpackungsmaterial.

Erfahrungsgemäß sind gewisse anwendungstechnische Anforderungen an Flüssigformulierungen für den Wasch-, Geschirrspül-, Reinigungs- und Körperreinigungsmittelbereich, wie z. B. eine hohe Lagerstabilität und gute Fließfähigkeit, mit einer konzentrierten Flüssigformulierung schwerer zu erzielen als mit einer verdünnten Form. Konzentrierte Flüssigformulierungen neigen im stärkeren Maße zu Vergelungen. Dies gilt vor allem für Alkylethersulfat- bzw. Alkylsulfat-haltige Flüssigformulierungen. So weisen z. B. marktgängige konzentrierte Universalreinigungsmittel einen Tensidanteil von höchstens 20 Gew.-% auf (s. J. Falbe, Surfactants in Consumer Products, S. 341, Springer Verlag, Berlin - Heidelberg - New York, 1987).

Geschirrspülmittelkonzentrate für den manuellen Bereich bestehen bis zu ca. 40 Gew.-% aus Tensiden (gleiche Quelle, S. 319). Eine Ausnahme bilden seifenhaltige Flüssigwaschmittel, die bis zu 50 Gew.-% Tenside (einschließlich der Seife) enthalten (s. H. Stache, H. Großmann, Waschmittel, S. 79, Springer Verlag, Berlin - Heidelberg - New York - London - Paris - Tokyo - Hongkong - Barcelona - Budapest, 1992). Der Anteil an Alkylethersulfaten bzw. Alkylsulfaten beträgt aus o. g. Gründen höchstens 10 Gew.-%. Erschwerend kommt hinzu, daß Alkylethersulfate bzw. Alkylsulfate bei Konzentrationen von ca. 30 - 55 Gew.-% sowie über 70 Gew.-% bzw. über 60 Gew.-% in Wasser so extrem hochviskos sind, daß sie in diesen - aus anwendungstechnischer Sicht außerordentlich interessanten - Konzentrationsbereichen bei den typischen Verarbeitungstemperaturen von bis zu 70 °C nur mit erheblichem technischen Aufwand, wenn überhaupt, weiterverarbeitet werden können. Wird die Temperatur insbesondere bei der Lagerung dieser Stoffe über einen längeren Zeitraum auf über 70 °C erhöht, so treten Zersetzungsprozesse ein.

EP 0 337 406 erwähnt die Herstellung von bis zu 60 Gew.-%igen niedrigviskosen Alkylethersulfat-Lösungen durch Zugabe von Neutralisationsmitteln zu den Alkyletherschwefelsäurehalbestern in Gegenwart von niedermolekularen Alkoholen wie Methanol, Ethanol oder Propanol als Lösevermittler in Wasser. Nachteil ist hier der niedrige Flammpunkt der verwendeten Alkohole. Beansprucht wird daher die Neutralisation in Wasser in Gegenwart eines Polyhydroxypolymers wie z. B. Polyglycerin oder hydriertem und hydrolysiertem Polysaccharid. Derart hergestellte Alkylethersulfat-Lösungen haben einen Aktivgehalt von mindestens 45 Gew.-%. Sie werden in Shampoo-Formulierungen eingesetzt, wo sie im Gegensatz zu dem in Gegenwart der angegebenen niedermolekularen Alkohole neutralisierten Alkyletherschwefelsäurehalbestern für eine erhöhte Viskosität sorgen. Derartig hergestellte Alkylethersulfate sind demzufolge nicht für die Herstellung konzentrierter und niedrigviskoser Wasch-, Geschirrspül-, Reinigungs- und Körperreinigungsmittelformulierungen geeignet.

US 4 477 372 beansprucht die Verwendung von fließfähigen und nichtwäßrigen Mischungen aus 40 bis 90 Gew.-% Alkylethersulfat- bzw. Alkylsulfat-Ammoniumsalz-Lösungen in 10 bis 60 Gew.-% nichtionischen Tensiden wie z. B. Fettalkohol- oder Nonylphenoloxethylaten als Lösemittel in Tensidformulierungen. Die fließfähigen, nichtwäßrigen Mischungen werden durch Neutralisation der entsprechenden Schwefelsäurehalbester mit sekundären bzw. tertiären Alkanolaminen in den nichtionischen Tensiden hergestellt.

Nachteil dieser Mischungen ist die durch die Anwesenheit eines bestimmten nichtionischen Tensids stark eingeschränkte Möglichkeit des Einsatzes in möglichst vielen verschiedenartigen Tensidformulierungen. So werden z. B. für den Wasch-, Geschirrspül-, Reinigungs- und Körperreinigungsmittelbereich je nach Erfordernis verschiedene nichtionische Tenside benötigt. Um diesem Umstand Rechnung zu tragen, müßten die die konzentrierten Alkylethersulfat- bzw. Alkylsulfat-Alkanolammoniumsalze enthaltenden Mischungen je nach Bedarf in verschiedenen nichtionischen Tensiden hergestellt werden. Dies ist im hohen Maße unökonomisch.

Flüssige Konzentrate von Alkylethersulfat- bzw. Alkylsulfat-Alkanolammoniumsalz-haltigen Mischungen aus nichtionischen Tensiden und Copolymeren aus Ethylen- und Propylenoxid sind ebenfalls beschrieben (JA 4848509, Abstract). Der Einsatz von Lösemittelmischungen muß ebenfalls als unwirtschaftlich angesehen werden. Auch schränkt hier die Anwesenheit eines bestimmten nichtionischen Tensids die Verwendung des Konzentrats wesentlich ein. JA 2045607 (Abstract) beschreibt konzentrierte Mischungen aus Alkylethersulfat- bzw. Alkylsulfatsalzen und Ampholyten in Lösemitteln wie Ethanol oder 1,2-Propandiol. Hier ist die Verwendung durch Anwesenheit des Ampholyten sowie des Lösemittels stark eingeschränkt.

Aufgabe der Erfindung war es daher, Alkylethersulfat- und/oder Alkylsulfat-haltige Konzentrate als Basistenside für Flüssigformulierungen zu entwickeln, die folgendes Anforderungsprofil erfüllen:
- Die Konzentrate sollen so niedrigviskos sein, daß sie bei Temperaturen bis zu 70 °C leicht zu konzentrierten homogenen und fließfähigen Flüssigformulierungen weiterverarbeitet werden können.
- Die Konzentrate sollen neben den Alkylethersulfat- bzw. Alkylsulfatsalzen keine weiteren Tenside und Lösemittel enthalten, um das Verwendungsspektrum möglichst breit zu halten.
- Die aus den Basistensiden herstellbaren konzentrierten Flüssigformulierungen sollen als Wasch-, Geschirrspül-, Reinigungs- und Körperreinigungsmittel einsetzbar sein und das Wasch- und Reinigungsvermögen herkömmlicher Flüssigformulierungen erreichen oder übertreffen.

Überraschenderweise wurde gefunden, daß die wasser- und lösemittelfreie Neutralisation der Alkylether- und Alkylschwefelsäurehalbester mit der nötigen Menge Alkanolaminen zu konzentrierten, unter der Einwirkung der Erdanziehungskraft zu bei höchstens 70 °C fließfähigen, Konzentraten führt, die als Basistenside für konzentrierte Flüssigformulierungen verwendet werden können. Mit "wasser- und lösemittelfrei" wird im Gegensatz zu den zum Stand der Technik gehörenden stark wasser- und/oder lösemittelhaltigen flüssigen Tensiden ein Wasser- und Lösemittelgehalt von 0 bis 10 Gew.-%, bevorzugt 0 bis 5 Gew.-% und besonders bevorzugt 0 bis 1 Gew.-%, bezeichnet.

Die Erfindung betrifft daher die Verwendung von wasser- und lösemittelfreien Tensidkonzentraten, enthaltend Mono-, Di- bzw. Trialkanolammoniumsalze der Alkylethersulfate und/oder Alkylsulfate hergestellt aus Verbindungen der Formel I und/oder Formel II

I R₁-O(C₂H₄O)ₘ-SO₃H

II R₁-O-SO₃H

mit
- R₁ =: C₁₀-C₂₀-Alkyl, wobei der Alkylrest linear oder verzweigt, gesättigt oder ungesättigt sein kann und
- m =: 1 - 7 bedeutet,
und der für die vollständige Neutralisation von I und/oder II benötigten Menge einer Verbindung der Formel III

III NR₂R₃R₄

mit
- R₂: = H oder C₂-C₄-Hydroxyalkyl
- R₃: = H oder C₂-C₄-Hydroxyalkyl
- R₄: = C₂-C₄-Hydroxyalkyl
als Basistenside für konzentrierte Flüssigformulierungen.

Die Herstellung der Schwefelsäurehalbester der Formel I oder II erfolgt in an sich bekannter Weise (s. S. Shore, D. R. Berger, Surfactants Science Series, Vol. 7, Part I, S. 135 ff., Marcel Dekker, Inc., New York and Basel, 1976) durch Sulfierung der entsprechenden Fettalkohole bzw. Fettalkoholoxethylate. Die Kohlenstoffkette der Alkohole kann linear oder verzweigt, gesättigt oder ungesättigt sein und 10 - 20 Kohlenstoffatome enthalten. Die Neutralisation erfolgt mit mindestens der stöchiometrisch erforderlichen Menge des Amins der Formel III. Bevorzugt wird ein Überschuß des Amins von bis zu 5 Gew.-% eingesetzt. Die Neutralisationstemperatur soll 70 °C nicht überschreiten. Die bevorzugte Neutralisationstemperatur beträgt 20 bis 50 °C.

Sollen Mischungen von Alkylethersulfaten und Alkylsulfaten hergestellt werden, so werden die Verbindungen gemäß Formel I und II vor der Neutralisation im gewünschten Verhältnis miteinander gemischt. Das Gemisch der Verbindungen gemäß Formel I und II kann aber auch in einer Weise hergestellt werden, bei der die entsprechenden Fettalkoholoxethylate und Fettalkohole gemeinsam sulfiert werden.

Die beanspruchten Tensidkonzentrate aus Alkylethersulfat- und/oder Alkylsulfat-Mono-, Di- bzw. Tri-alkanolammoniumsalzen stellen hervorragende Ausgangsmaterialien zur Herstellung konzentrierter Flüssigformulierungen für den Wasch-, Geschirrspül-, Reinigungs- und Körperreinigungsmittelbereich dar. Die konzentrierten Flüssigformulierungen können weitere übliche Zusätze wie z. B. andere Tenside, pH-Regulatoren, Viskositätsregulatoren, Farb- und Geruchsstoffe, Konservierungsmittel, organische Lösemittel und Wasser enthalten. Durch Verdünnen mit Wasser oder organischen Lösemitteln können auch Flüssigformulierungen mit geringem Tensidgehalt hergestellt werden, jedoch ist dieses Vorgehen nicht bevorzugt. Der Gesamttensidgehalt kann auf diese Weise beliebig zwischen 1 und 100 Gew.-% eingestellt werden, wobei Gesamttensidgehalte von 5 bis 80 Gew.-% bevorzugt werden. Besonders bevorzugt wird der Bereich von 20 bis 70 Gew.-% Gesamttensidgehalt. Der Anteil der Basistenside an den Gesamttensiden beträgt 5 bis 100 %. Die nachfolgenden Beispiele sollen die erfindungsgemäßen Ansprüche belegen, ohne diese einzuschränken.

### Beschreibung der anwendungstechnischen Untersuchungsmethoden

### Bestimmung des Reinigungsvermögens von Geschirrspülmittelformulierungen für den manuellen Bereich

Die Beurteilung des Reinigungsvermögens von Spülmittelformulierungen erfolgt durch Spültests mit angeschmutzten Tellern. Der sogenannte Miniteller-Spültest (s. R. M. Anstett et al., JAOCS 43 (1966), S. 576 ff.) wird wie folgt durchgeführt: In einer Kristallisierschale (⌀ 13,5 cm) werden 400 ml Tensidlösung (Tensidgehalt 0,075 g/l, 12,5° dH) auf 50 °C erwärmt. In der Tensidlösung werden große und kleine Uhrgläser (⌀ 4,0 bzw. 2,9 cm) gespült, die mit 0,36 bzw. 0,12 g Schweineschmalz angeschmutzt sind. Das Reinigen erfolgt unter Bürsten und Schaumschlagen mit einem Rindshaarpinsel bis zum Schaumzusammenbruch. Die Spülleistung wird durch die sogenannte Tellerzahl ausgedrückt. Dabei zählt das große Uhrglas drei Tellereinheiten, das kleine eine Tellereinheit. Vergleichend wurde ein marktgängiges Geschirrspülmittel für den manuellen Bereich ("Pril", Hersteller Fa. Henkel, UBA-Nr. 04161461, Tensidgehalt 28 Gew.-%) dem gleichen Test unterzogen.

### Bestimmung des Fett-/Ölaufnahmevermögens von Geschirrspülmittelformulierungen für den manuellen Bereich

Die Öltitrationsmethode dient zur Bestimmung des Emulgier- bzw. Fettaufnahmevermögens von Spülmitteln. Ein 1 000 ml-Becherglas wird mit 500 ml Trinkwasser von 45 °C gefüllt und ein ESGE-Haushaltsrührer mit der Emulgierrührscheibe 0,5 cm unter der Wasseroberfläche eingestellt. In das Becherglas werden der 0,125 g/l Tensidgehalt entsprechenden Menge des Spülmittels gegeben und 30 s schaumig gerührt. Anschließend werden 3 ml Ölivenöl zugetropft und nochmals 30 s gerührt. Danach wird 90 s ruhengelassen. Die weitere Ölzugabe erfolgt ml-weise. Wenn nach der Ruhezeit kein Schaum oder nur am Rand eine schmale Schaumperlenkette vorhanden ist, ist der Endpunkt erreicht. Vergleichend wurde ein marktgängiges Geschirrspülmittel für den manuellen Bereich ("Pril", Hersteller Fa. Henkel, UBA-Nr. 04161461, Tensidgehalt 28 Gew.-%) dem gleichen Test unterzogen.

### Bestimmung des Waschvermögens von Flüssigwaschmitteln

Das Waschvermögen der zu untersuchenden Flüssigformulierung wird im Vergleich zu einer herkömmlichen Flüssigformulierung (im folgenden Rahmenrezeptur genannt) folgender Zusammensetzung bestimmt:

Rahmenrezeptur (Vergleichsrezeptur) (Tensidgehalt 33,3 Gew.-%)
5,3 Gew.-% C₁₂-C₁₆-Alkylsulfat-Natrium-Salz
17,7 Gew.-% Kokosfettsäure-Natrium-Salz
10,3 Gew.-% C₁₂-C₁₄-Alkylalkohol-9 mol EO/mol-Addukt
12,0 Gew.-% Ethanol
Parfüm, Konservierungsmittel, Wasser zu 100 Gew.-%.

Zur Bestimmung des Waschvermögens werden Waschversuche unter folgenden Bedingungen durchgeführt:
- Waschtemperatur:: 30 °C für Polyester (PE)- und Mischgewebe (MG),
60 °C für Baumwolle (BW) und MG,
95 °C für BW.
- Waschmaschine:: AEG Öko-Lavamat 945 für Waschtemperaturen von 60 °C und 95 °C.
AEG Öko-Lavamat 610 für die Waschtemperatur von 30 °C.
- Waschmitteldosierung:: Von der Rahmenrezeptur werden 120 g pro Waschgang eingesetzt. Von den entwickelten konzentrierten Flüssigformulierungen wird entsprechend dem Tensidgehalt weniger eingesetzt.
- Wasserhärte:: 12° dH.

Die testangeschmutzten Prüfläppchen (11 cm x 18 cm, WFK-Institut, Krefeld) werden auf ein Frotteehandtuch genäht (jeweils ein Doppelversuch) und zusammen mit Ballastgewebe (1 kg bei 30 °C, 3 kg bei 60 °C und 95 °C) gewaschen. Die Waschwerte werden spektralphotometrisch ermittelt, wobei die Bestimmung relativ zu einem Weißstandard des WFK-Instituts (≙ 100 %) und einer Nullprobe (ungewaschenes Testgewebe, ≙ 0 %) mit Hilfe eines Datacolor-Spektralphotometers (Programm FM 81C) bei X = 560 nm erfolgt (2fach Bestimmung).

### Bestimmung des Reinigungsvermögens von Universalreinigern mit Hilfe des Scheuertests

Die Bestimmung des Reinigungsvermögens erfolgt in Anlehnung an die in SÖFW 112 (1986), S. 371 ff. beschriebenen Prüfung. Als Testanschmutzung wird folgendes Gemisch verwendet:
7 Gew.-% Spezialschwarz 4 (Fa. Degussa)
17 Gew.-% Myritol 318 (Fa. Henkel)
40 Gew.-% Telura 310 (Fa. Esso)
36 Gew.-% Benzin 80/110.

Das Reinigungsvermögen wird mit Hilfe eines Datacolor-Spektralphotometers, (Programm FM 81C) bei X = 560 nm ermittelt (2fach Bestimmung). In dem Scheuertest wird jeweils die Menge der Universalreinigerformulierung eingesetzt, die einem Tensidgehalt von 1 Gew.-% entspricht. Vergleichend wird eine Rahmenrezeptur mit üblicher Tensidkombination und dem gleichen Tensidgehalt wie der der zu entwickelnden konzentrierten Formulierungen dem gleichen Test unterzogen. Die Rahmenrezeptur hat folgende Zusammensetzung:
19,5 Gew.-% C₁₃-C₁₇-sek. Paraffinsulfonat-Natrium-Salz
5,0 Gew.-% C₁₂-C₁₄-Alkoholethersulfat-2 mol EO/mol-Natrium-Salz
3,0 Gew.-% Fettsäureamidopropylbetain
Rest zu 100 Gew.-% Wasser.

Der Tensidgehalt beträgt 27,5 Gew.-%.

### Bestimmung des Lagerverhaltens

Zur Bestimmung des Lagerverhaltens der konzentrierten Flüssigformulierungen werden diese bei 4 °C gelagert. Alle 4 Tage erfolgt eine visuelle Begutachtung. Die maximale Lagerzeit beträgt 28 Tage.

### Bestimmung des Klarschmelzpunktes

Die konzentrierten Flüssigformulierungen werden eingefroren und anschließend langsam erwärmt. Als Klarschmelzpunkt wird die Temperatur bezeichnet, bei der die Flüssigformulierung vom trüben in den klaren Zustand übergeht.

### Bestimmung der Viskosität

Von den konzentrierten Alkylethersulfat- bzw. Alkylsulfat-Mono-, Di- bzw. Trialkanolammonium-Salzen bzw. den diese Substanzen enthaltenen Flüssigformulierungen wird die Viskosität mit Hilfe eines Haake-Rheometers (NV-DIN bzw. SV-DIN) bei einem Schergefälle von 2·s⁻¹ bei 20 °C bestimmt.

### Eingesetzte Substanzen

- MARLON® PS 65:: C₁₃-C₁₇-Sek. Paraffinsulfonat-Natrium-Salz, Aktivgehalt 65 Gew.-% in Wasser.
- AMPHOLYT JB 130:: Fettsäureamidopropylbetain, Aktivgehalt 30 Gew.-% in Wasser.
- MARLOX® FK 64:: C₁₀-C₁₂-Fettalkohol-EO-PO-Additionsprodukt.
- MARLIPAL® 24/20:: C₁₂-C₁₄-Fettalkohol-2 mol EO/mol-Addukt.
- MARLIPAL® 24/90:: C₁₂-C₁₄-Fettalkohol-9 mol EO/mol-Addukt.
- Probe 1:: C₁₂-C₁₄-Fettalkoholethersulfat-2 mol EO/mol-Monoethanolammonium-Salz.
- Probe 2:: C₁₂-C₁₄-Fettalkoholethersulfat-2 mol EO/mol-Triethanolammonium-Salz.
- Probe 3:: C₁₂-C₁₄-Fettalkoholsulfat-Triethanolammonium-Salz.
- Probe 4:: C₁₂-C₁₄-Fettalkoholethersulfat-2 mol EO/mol-C₁₂-C₁₄-Fettalkoholsulfat-Monoethanolammonium-Salze - 1 : 1-Gemisch.
- Probe 5:: C₁₂-C₁₄-Fettalkoholethersulfat-2 mol EO/mol-C₁₂-C₁₄-Fettalkoholsulfat-Triethanolammonium-Salze - 1 : 1-Gemisch.
- Probe 6:: C₁₂-C₁₄-Fettalkoholethersulfat-2 mol EO/mol-Monoisopropanolammonium-Salz.
- Probe 7:: C₁₂-C₁₄-Fettalkoholethersulfat-2 mol EO/mol-Diisopropanolammonium-Salz.
- Probe 8:: C₁₂-C₁₄-Fettalkoholethersulfat-2 mol EO/mol-Triisopropanolammonium-Salz.

® = Eingetragenes Warenzeichen der HÜLS AKTIENGESELLSCHAFT.

Bei den erfindungsgemäßen Proben 1 bis 8 handelt es sich um bei 70 °C unter Einwirkung der Erdanziehungskraft fließfähige Tensidkonzentrate.

### Beispiel 1:

Die nachstehend aufgeführte Tabelle gibt einen Überblick über Zusammensetzung und anwendungstechnische Eigenschaften einer beispielhaft ausgewählten konzentrierten Flüssigformulierung für den Geschirrspülmittelbereich (manuell) (Angaben in Gew.-% bezogen auf Aktivgehalt).

| Bestandteil | Formulierung 1 (erfindungsgemäß) | Vergleichsformulierung Pril |
|---|---|---|
| Probe 6 | 14,1 | |
| MARLON PS 65 | 32,9 | |
| AMPHOLYT JB 130 | 3,0 | |
| 1,2-Propandiol | 6,2 | |
| Harnstoff | 2,5 | |
| Ethanol | 5,0 | |
| Parfüm, Farbstoff, | | |
| Wasser | Rest zu 100 | |
| Tensidanteil | 50 | 28 |
| Klarschmelzpunkt [°C] | 4 | 3 |
| Ein-Teller-Test-Einheiten | 27 | 27 |
| Ölaufnahmevermögen [ml] | 7,75 | 7,50 |

Formulierung 1 stellt ebenso wie Pril ein leistungsfähiges Geschirrspülmittel dar, wobei dessen Tensidanteil fast doppelt so hoch liegt wie der von Pril.

### Beispiel 2:

Die folgende Tabelle gibt einen Überblick über Zusammensetzung und anwendungstechnische Eigenschaften beispielhaft ausgewählter konzentrierter Flüssigwaschmittelformulierungen (Angaben in Gew.-% bezogen auf Aktivgehalt).

| Bestandteil | | Formulierung 2 (erfindungsgemäß) | Formulierung 3 (erfindungsgemäß) | Rahmenrezeptur |
|---|---|---|---|---|
| Probe 6 | | 30,0 | - | |
| Probe 8 | | - | 30,0 | |
| MARLIPAL 24/20 | | 2,0 | 2,0 | |
| MARLOX FK 64 | | 17,5 | 17,5 | |
| Kokosfettsäure | | 15,0 | 15,0 | |
| Monoethanolamin | | 5,5 | 9,3 | |
| Polyethylenglykol 600 | | 13,1 | 5,0 | |
| 1,2-Propandiol | | - | 12,8 | |
| Ethanol | | 10,0 | 8,8 | |
| Wasser | | | Rest zu 100 | |
| Tensidanteil | | 66,0 | 66,0 | 33,3 |
| Klarschmelzpunkt [°C] | | 3 | 1 | 4 |

| Waschwert [%] | | | | |
|---|---|---|---|---|
| bei 30 °C | PE | 37,6 | 42,0 | 32,4 |
| | MG | 61,3 | 65,7 | 58,7 |
| bei 60 °C | MG | 63,7 | 65,6 | 56,8 |
| | BW | 59,9 | 58,8 | 59,1 |
| bei 95 °C | BW | 64,9 | 69,4 | 68,8 |

Die Formulierungen 2 und 3 mit einem Tensidgehalt von jeweils 66 Gew.-% stellen Hochkonzentrate mit einem hohen Leistungsvermögen dar. Sie zeigen gleiches bzw. sogar höheres Waschvermögen als die herkömmliche Rahmenrezeptur, die als repräsentativ für marktgängige Flüssigwaschmittel angesehen werden kann.

### Beispiel 3:

Die folgende Tabelle gibt einen Überblick über Zusammensetzung und anwendungstechnische Eigenschaften beispielhaft ausgewählter konzentrierter Flüssigformulierungen für Universalreiniger (Angaben in Gew.-% bezogen auf Aktivgehalt).

| Bestandteil | Formulierung 4 (erfindungsgemäß) | Formulierung 5 (erfindungsgemäß) | Rahmenrezeptur |
|---|---|---|---|
| Probe 6 | 13,1 | - | |
| Probe 8 | - | 14,8 | |
| MARLON PS 65 | 11,4 | 9,8 | |
| AMPHOLYT JB 130 | 3,0 | 3,0 | |
| K-Na-Cumol-Sulfonat | 5,0 | 5,0 | |
| 1,2-Propandiol | 5,7 | 6,3 | |
| Wasser | | Rest zu 100 | |
| Tensidanteil | 27,5 | 27,5 | 27,5 |
| Klarschmelzpunkt [°C] | 2 | 2 | 4 |
| Viskosität [mPa·s] | < 100 | < 100 | 1 000 |
| Reinigungsvermögen [%] | 61,2 | 61,8 | 60,3 |

Die Formulierungen 4 und 5 stellen Universalreiniger mit einem hohen Reinigungsvermögen dar. Trotz deren hoher Tensidanteile handelt es sich um niedrigviskose Flüssigkeiten.

## Patentansprüche

1. Verwendung von wasser- und lösemittelfreien Tensidkonzentraten, enthaltend Mono-, Di- bzw. Trialkanolammoniumsalze der Alkylethersulfate und/oder Alkylsulfate hergestellt aus Verbindungen der Formel I und/oder Formel II
I R₁-O(C₂H₄O)ₘ-SO₃H
II R₁-O-SO₃H
mit
R₁ = C₁₀-C₂₀-Alkyl, wobei der Alkylrest verzweigt oder linear, gesättigt oder ungesättigt sein kann und
m = 1 - 7
bedeutet,
und der die für die vollständige Neutralisation von I benötigten Menge einer Verbindung der Formel III
III NR₂R₃R₄
mit
R₂ = H oder C₂-C₄-Hydroxyalkyl
R₃ = H oder C₂-C₄-Hydroxyalkyl
R₄ = C₂-C₄-Hydroxyalkyl
als Basistensid für konzentrierte Flüssigformulierungen.

2. Verwendung der Tensidkonzentrate nach Anspruch 1 als Basistensid in konzentrierten Flüssigformulierungen, wobei der Anteil der Basistenside an den Tensiden 5 bis 100 % beträgt.

3. Verwendung der Tensidkonzentrate nach den Ansprüchen 1 und 2 als Basistensid für konzentrierte Flüssigwaschmittel.

4. Verwendung der Tensidkonzentrate nach den Ansprüchen 1 und 2 als Basistensid für konzentrierte flüssige manuelle Geschirrspülmittel.

5. Verwendung der Tensidkonzentrate nach den Ansprüchen 1 und 2 als Basistensid für konzentrierte flüssige Universalreinigungsmittel harter Oberflächen.

6. Verwendung der Tensidkonzentrate nach den Ansprüchen 1 und 2 als Basistensid für konzentrierte flüssige Körperreinigungsmittel.
